(19) Europäisches Patentamt
European Patent Office
Office européen des brevets

(11) Veröffentlichungsnummer: **0 071 808**
**B1**

# (12) EUROPÄISCHE PATENTSCHRIFT

(45) Veröffentlichungstag der Patentschrift: **25.09.85**

(51) Int. Cl.⁴: **C 07 C 143/18,** C 07 C 139/00

(21) Anmeldenummer: **82106467.2**

(22) Anmeldetag: **19.07.82**

(54) **Verfahren zur Herstellung von alpha-ständig sulfonierten, alpha, beta-ungesättigten Carbonsäurealkylestern sowie danach erhältliche Verbindungen.**

(30) Priorität: **29.07.81 DE 3129980**

(43) Veröffentlichungstag der Anmeldung: **16.02.83 Patentblatt 83/7**

(45) Bekanntmachung des Hinweises auf die Patenterteilung: **25.09.85 Patentblatt 85/39**

(84) Benannte Vertragsstaaten: **DE FR GB IT**

(56) Entgegenhaltungen:
**FR - A - 2 110 792**
**US - A - 2 743 288**
**US - A - 2 895 987**

**Chemical Abstracts Band 79, Nr. 23, 10. Dezember 1973, Columbus, Ohio, USA, A. LE BERRE et al. "Alpha-Sulfocarboxylic acids and derivatives. III. Sulfonation and chlorosulfonylation of acrylic and 3-chloropropionic acids", Seite 324, Spalte 2, Abstract Nr. 136418f**

(73) Patentinhaber: **BAYER AG, Konzernverwaltung RP Patentabteilung, D-5090 Leverkusen 1 Bayerwerk (DE)**

(72) Erfinder: **Neukam, Theo, Dr., Rubensstrasse 14, D-4047 Dormagen (DE)**
Erfinder: **Bräuer, Wolfgang, Dr., Mozartstrasse 43, D-5090 Leverkusen 1 (DE)**
Erfinder: **Korte, Siegfried, Dr., Bamberger Strasse 6, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 071 808 B1

**Beschreibung**

Die vorliegende Erfindung betrifft ein Verfahren zur Herstellung sulfonierter, $\alpha,\beta$-ungesättigter Carbonsäurealkylester der allgemeinen Formel

$$R_1CH=C\genfrac{}{}{0pt}{}{CO_2R_2}{SO_3X} \qquad (I)$$

in der

$R_1$ Wasserstoff oder Methyl,

$R_2$ eine primäre oder sekundäure Alkylgruppe mit 1 bis 4 C-Atomen und

X Wasserstoff oder eine primäre oder sekundäre Alkylgruppe mit 1 bis 4 C-Atomen

bedeuten, sowie deren Sulfonsäuresalze.

Die Erfindung betrifft weiterhin Verbindungen der Formel

$$R_1\text{-}CH=C\genfrac{}{}{0pt}{}{CO_2R_2}{SO_3^{\ominus}\ X^{\oplus}} \qquad (Ia)$$

worin $R_1$ und $R_2$ die vorstehend genannte Bedeutung aufweisen und

$X^{\oplus}$ ein Proton oder ein Metallionäquivalent bedeuten, wobei als Metalle Alkali- und Erdalkalimetalle, Zink und Aluminium bevorzugt sind.

Es ist bereits bekannt, $\alpha,\beta$-ungesättigte Carbonsäuren durch Umsetzung mit Sulfonierungsreagenzien in Sulfonsäurederivate zu überführen [Rec. Trav. Chim. 62, 46 (1943); US-PS 2 895 987; FR-PS 2 110 792, Bull. Soc. Chim. Fr. 1973, 2266], wobei die 2-Sulfoacrylsäure aber bisher nur in verdünnter Lösung hergestellt wurde, da sie beim Einengen der Lösung polymerisiert. Auch der Versuch, die 2-Sulfoacrylsäure durch Sulfonierung mit reinem Schwefeltrioxid zu isolieren, schlug fehl.

Weiterhin wird beschrieben, dass die aus der Acrylsäure durch Sulfonierung mit Chlorsulfonsäure gewonnene 3-Chlor-2-sulfopropionsäure mit Chlormethylformiat nach 18 Stunden bei 95°C den 2--Methoxysulfonylpropensäure-methylester ergibt (FR-PS 2 110 792). Durch Veresterung mit Methanol, Umsetzung mit Pyridin zum Pyridiniumsalz und anschliessender Eliminierung durch Dimethyllaurylamin wird aus 3-Chlor-2-sulfopropionsäure das sehr hygroskopische Dimethyllauryl-ammoniumsalz des 2-Sulfopropensäuremethylesters erhalten (Bull. Soc. Chim. Fr. 1973, 2266). Nachteilig bei diesen vorbeschrieben Verfahren ist, dass die technisch besonders interessanten, von den Carbonsäuren und/oder Sulfonsäuren abgeleiteten Esterderivate nicht oder nur über aufwendige mehrstufige Synthesewege zugänglich sind. Eine technische Anwendung dieser komplizierten Verfahren ist nicht möglich.

Es wurde nun ein Verfahren zur Herstellung von Verbindungen der Formel I gefunden, das dadurch gekennzeichnet ist, dass man $\alpha,\beta$-ungesättigte Carbonsäureester der allgemeinen Formel

$$R_1CH=CH\text{-}CO_2R_2 \qquad (II)$$

in der $R_1$ und $R_2$ die obengenannten Bedeutungen haben, bei Temperaturen von —10°C bis +25°C mit 0,6 bis 1,6 Moläquivalenten Schwefeltrioxid und gegebenenfalls mit 0,1 bis 10 Moläquivalenten eines Alkylierungsmittels, jeweils bezogen auf den ungesättigten Ester, umsetzt, das Reaktionsgemisch 0,5 bis 6 Stunden auf Temperaturen von 70°C bis 200°C erhitzt, destillativ aufarbeitet und die Komponenten des Destillats gegebenenfalls trennt. Die destillative Aufarbeitung erfolgt, gegebenenfalls nach Abtrennung leicht flüchtiger Bestandteile, z.B. nicht umgesetztem Alkylierungsmittel, insbesondere so, dass man die Destillation in einem Verdampfungsrohr bei Temperaturen von 100°C bis 250°C, einem Druck von 0,05 bis 30 mbar und einem Durchsatz von 0,1 bis 10 kg/m².h kontinuierlich betreibt. Das Destillat, überwiegend bestehend aus 5 - 90 Gew.-% der Sulfonsäure I (X = H) und 10 - 95 Gew.-% des Sulfonsäureesters I ($X = R_2$) wird, durch Ausfällung der Sulfonsäure mit einem organischen Fällmittel oder durch Salzbildung aufgetrennt. Der verbleibende Sulfonsäureester wird destillativ isoliert.

Als $\alpha,\beta$-ungesättigte Carbonsäurealkylester kommen z.B. Acrylsäuremethylester, Acrylsäureethylester, Acrylsäurepropylester, Acrylsäureisopropylester, Acrylsäurebutylester, Acrylsäureisobutylester, Crotonsäuremethylester, Crotonsäureethylester, Crotonsäurepropylester, Crotonsäureisopropylester, Crotonsäurebutylester und Crotonsäureisobutylester in Frage. Vorzugsweise werden Acrylsäuremethylester Acrylsäureethylester und Crotonsäuremethylester benutzt.

Vorzugsweise wird mit 0,8 bis 1,2 Moläquivalenten Schwefeltrioxid, das gasförmig oder in flüssiger Form unter Rühren eingeleitet werden kann, umgesetzt. Die Dosierung des Schwefeltrioxids soll möglichst schnell erfolgen. Es ist jedoch zu beachten, dass die genannte Umsetzungstemperatur, vorzugsweise 0 bis +10°C, eingehalten wird. Die Dosierdauer ist daher von der Grösse und Kühlkapazität des Ansatzes abhängig. In der Regel erstreckt sich die Zugabe des $SO_3$ auf einen Zeitraum von 0,1 bis 2,0 Stunden. Es kann bei stark exothermen Reaktionen, zum Beispiel bei Verwendung von Crotonsäuremethylester, zweckmässig sein, die Umsetzung in Gegenwart eines organischen Lösungsmittels, insbesondere eines niedrig siedenden Chlorkohlenwasserstoffs, wie Chloroform oder Methylenchlorid, welches bis zu 100 Gew.-%, bezogen auf eingesetzten Carbonsäureester und Schwefeltrioxid, zugesetzt wird, durchzuführen. Im Regelfall, vor allem bei Acrylsäuremethylester, wird das Schwefeltrioxid ohne Lösungsmittel zugegeben.

Als Alkylierungsmittel kommen z.B. in Frage: $C_1$-$C_4$-Dialkylsulfate, Alkyl- und Aryl-Alkyl-Ether, Alkohole, Alkylhalogenide und Diazomethan. Bevorzugt wird, insbesondere bei Einsatz von Acrylsäuremethylester als $\alpha,\beta$-ungesättigter Carbonsäureester, Dimethylsulfat.

Vorzugsweise setzt man 0,2 bis 5 Moläquivalente $C_1$-$C_4$-Alkylsulfat, bezogen auf den ungesättigten Ester, ein.

Die Reaktion erfolgt üblicherweise chargenweise in einem Rührreaktor, kann aber auch kontinuierlich in einem Reaktionsrohr unter gleichzeitiger Zuleitung der Reaktionskomponenten geführt werden.

Das Alkylierungsmittel wird nach oder während der Umsetzung mit Schwefeltrioxid zugegeben. Danach wird das Reaktionsgemisch vorzugsweise 1,5 bis 3 Stunden auf 80 bis 160°C erhitzt.

Die kontinuierliche Destillation kann in einem Verdampfungsrohr durchgeführt werden. Vorzugsweise wird ein Dünnschichtverdampfer mit rotierendem Wischer oder ein Fallfilmverdampfer benutzt. Bei dieser kontinuierlichen Destillation beträgt die Temperatur vorzugsweise 140°C bis 200°C und der Druck vorzugsweise 0,3 bis 3 mbar. Die Verweilzeit im Verdampfer liegt bei 0,1 bis 10 kg Produkt pro $m^2$ Austauschfläche des Verdampfungsrohres und pro Stunde.

Das andallende Reaktionsgemisch besteht überwiegend aus der Sulfonsäure und dem Sulfonsäureester der Formel I. Im Fall der Reaktion ohne Zusatz eines Alkylierungsmittels dominiert die Sulfonsäure, im Fall der Reaktion mit Zusatz eines Alkylierungsmittels überwiegt der Sulfonsäureester.

Die Ausbeute des Reaktionsgemisches, bezogen auf die eingesetzten Ausgangsverbindungen, beträgt 50 - 100 Gew.-%.

Als Fällmittel für die Isolierung der Sulfonsäure der Formel I mit X = Wasserstoff aus dem Gemisch wird ein organisches Lösungsmittel benutzt, vorzugsweise ein aromatischer Kohlenwasserstoff, z.B. Benzol, Toluol, Xylol. Eine besonders gute Reinigungswirkung wird erzielt, wenn man das Gemisch mit dem Fällmittel kurz aufheizt und dann abkühlen lässt, wobei die Sulfonsäure ausfällt.

Das nach der Abtrennung der Sulfonsäure verbliebene Filtrat, in dem hauptsächlich das organische Lösungsmittel und der Sulfonsäureester sind, wird destillativ von dem organischen Lösungsmittel befreit. Die anschliessende fraktionierte Destillation über eine Kolonne bei einer Temperatur von 70°C bis 200°C, vorzugsweise 80°C bis 150°C, und einem Druck von 0,05 bis 40 mbar, vorzugsweise 0,3 bis 7 mbar, ergibt den Sulfonsäureester der Formel I mit $X = R_2$.

Eine weitere Möglichkeit zur Abtrennung der Sulfonsäure besteht in der Salzbildung mit den Carbonaten, Oxiden oder Acetaten von Alkali-, Erdalkalimetallen, Aluminium und Zink, vorzugsweise mit ZnO, MgO, Na-acetat. Sie wird in polaren organischen Lösungsmitteln, vorzugsweise in Acetonitril oder Essigsäure, durchgeführt. Aus dem Filtrat kann nach der schon oben beschriebenen Verfahrensweise der Sulfonsäureester destillativ gewonnen werden.

Die nach dem erfindungsgemässen Verfahren hergestellten Produkte sind wertvolle organische Zwischenprodukte. Durch Anlagerung nucleophiler Agentien an die Doppelbindung sind zum Beispiel oberflächenaktive Substanzen sowie Mittel mit mikrozider und fungizider Wirkung zugänglich.

*Beispiel 1*

Umsetzung von Acrylsäuremethylester mit $SO_3$

a) *Herstellung eines Gemisches aus 2-Sulfo-propensäuremethylester und 2-Methoxysulfonyl-propensäuremethylester*

In eine Vorlage von 506 g Acrylsäuremethylester, stabilisiert mit 2 g Hydrochinon, werden bei 0 bis 10°C unter Einleiten eines schwachen Inertgas-Stromes 471 g Schwefeltrioxid in flüssiger Form eingerührt. Nach 2stündigem Tempern des primär entstandenen Gemisches bei 100°C werden im Vakuum leicht flüchtige Bestandteile — überwiegend nicht umgesetzter Acrylsäuremethylester und bei der Umsetzung anfallendes Dimethylsulfat — abdestilliert. Anschliessend wird das vorliegende Rohgemisch unter folgenden Bedingungen in einen Dünnschichtverdampfer eingetragen:

| | |
|---|---|
| Temperatur des Heizmantels: | 158°C |
| eingestellter Druck: | 0,5 bis 1,0 mbar |
| Durchsatzmenge: | 1,0 kg/$m^2$h |

Man erhält als Kondensat ein nahezu farbloses Öl, bestehend aus 2-Sulfopropensäuremethylester und 2-Methoxysulfonyl-propensäuremethylester, aus dem sich die Säure teilweise als kristalline Phase ausscheidet.

Ausbeute: 622 g (64 Gew.-%, bezogen auf die eingesetzten Komponenten Acrylsäuremethylester und $SO_3$).

NMR-spektroskopisch lässt sich die folgende Zusammensetzung ableiten:

60 Gew.-% 2-Sulfo-propensäuremethylester und 40 Gew.-% 2-Methoxysulfonyl-propensäuremethylester.

b) *Isolierung von 2-Sulfo-propensäuremethylester*

Das bei der Dünnschichtdestillation anfallende, aus Sulfonsäure und Sulfonsäureester bestehende Gemisch wird unter kurzzeitigem Aufheizen mit 500 g Benzol verrührt. Nach Abkühlen scheidet sich der 2-Sulfo-propensäuremethylester mit hoher Reinheit in kristalliner Form ab.

Ausbeute: 200 g (19,5 Gew.-%, bezogen auf die eingesetzten Komponenten Acrylsäuremethylester und $SO_3$)

Schmelzpunkt: 66°C

$^1$H-NMR-Spektrum (in $d_3$-Acetonitril): $\delta$ 3,85 (s), 6,85 (d), 6,9 (d), 11,0 (s) ppm

c) *Isolierung von 2-Methoxysulfonyl-propensäuremethylester*

Das nach Ausfällung und Abtrennen der Sulfonsäure verbleibende Filtrat wird unter Abdampfen des Benzols eingeengt. Der Rückstand wird unter Fraktionierung destilliert. Die bei 93°C und 0,4 mbar übergehende Fraktion besteht aus 2-Methoxysulfonyl-propensäuremethylester.

Ausbeute: 108 g (10,5 Gew.-%, bezogen auf die eingesetzten Komponenten Acrylsäuremethylester und $SO_3$)

Schmelzpunkt: 25 - 30°C

$^1$H-NMR-Spektrum (in $d_3$-Acetonitril): $\delta$ 3,85 (s), 3,9 (s), 6,9 (d), 7,02 (d) ppm

d) *Isolierung des Na-Salzes des 2-Sulfo-propensäuremethylesters*

66,5 g des gemäss a) durch Dünnschichtdestillation gewonnenen Kondensatgemisches werden in 60 g Acetonitril gelöst. Bei Raumtemperatur werden nun unter Rühren 32,8 g Na-Acetat portionsweise eingetragen. Der sich ausscheidende weisse Feststoff wird durch Filtration abgetrennt. Nach Trocknung liegt das Na-Salz des 2-Sulfopropensäuremethylesters vor.

Ausbeute: 41,8 g (35 Gew.-%, bezogen auf eingesetzten Acrylsäuremethylester)

Schmelzpunkt: 210°C

$^1$H-NMR-Spektrum (in $D_2O$): $\delta$ 3,85 (s), 6,65 (d) ppm

Aus dem Filtrat kann nach der unter c) beschriebenen Methode der 2-Methoxysulfonyl-propensäuremethylester gewonnen werden.

*Beispiel 2* (nicht erfindungsgemäss)

Zu 180 g Acrylsäuremethylester, stabilisiert mit 2 g Hydrochinon, werden im Verlauf von 40 Minuten 154 g Schwefeltrioxid getropft. Dabei wird ein Temperaturanstieg auf 55°C zugelassen. Das anfallende Rohgemisch wird insgesamt aus einer Vorlage unter Zwischenschaltung einer kurzen Vigreux-Kolonne destilliert, derart, dass bei einem eingestellten Druck von 0,1 bis 1,0 mbar und bei von 60°C auf 180°C ansteigenden Temperaturen Fraktionen abgetrennt werden. Zunächst gehen bei 60 bis 100°C unter starkem Schäumen des vorgelegten Gemisches 26 g einer farblosen Flüssigkeit, bestehend aus Acrylsäuremethylester und Dimethylsulfat, über. Danach lässt sich nur noch eine bei 108 bis 112°C übergehende Fraktion abtrennen. Man erhält geringe Mengen eines farblosen Öls, das spektroskopisch als 2-Methoxysulfonyl-propensäuremethylester identifiziert wurde.

Ausbeute: 27 g (8,1 Gew.-%, bezogen auf die eingesetzten Komponenten Acrylsäuremethylester und $SO_3$)

*Beispiele 3 bis 11*

Die in der folgenden tabellarischen Übersicht aufgelisteten Beispiele beschreiben Variationsmöglichkeiten im Rahmen des erfindungsgemässen Verfahrens, die sich durch Änderung wesentlicher Verfahrensparameter sowie durch Einsatz andersartiger Esterkomponenten ergeben. Die Verfahrensführung entspricht Beispiel 1.

| Beispiele | 3 | 4 | 5 | 6 | 7 | 8 | 9[1)] | 10[2)] | 11 |
|---|---|---|---|---|---|---|---|---|---|
| Esterkomponente der Formel II | $R_1$:H $R_2$:$CH_3$ | $R_1$:H $R_2$:$CH_3$ | $R_1$:H $R_2$:$CH_3$ | $R_1$:H $R_2$:$CH_3$ | $R_1$:H $R_2$:$CH_3$ | $R_1$:H $R_2$:$CH_3$ | $R_1$:H $R_2$:$CH_3$ | $R_1$:$CH_3$ $R_2$:$CH_3$ | $R_1$:H $R_2$:$C_2H_5$ |
| Molverhältnis Ester : $SO_3$ | 1/0,65 | 1/1 | 1/1,6 | 1/1 | 1/1 | 1/1 | 1/1 | 1/1 | 1/1 |
| Verfahrensführung nach $SO_3$-Zugabe | | | | | | | | | |
| Temperatur [°C] | 100 | 100 | 100 | 100 | 80 | 150 | 100 | 100 | 100 |
| Dauer [Std.] | 1,0 | 0,5 | 1,0 | 5,0 | 1,0 | 3,0 | 2,0 | 2,0 | 2,0 |
| Rohgemisch-Ausbeute[3)] (nach Dünnschichten) [Gew.-%] | 34,0 | 63,0 | 47,0 | 59,0 | 46,0 | 64,0 | 61,0 | 45,0 | 40,0 |
| Sulfonsäure-Ausbeute[4)] (berechnet aus Anteil im Rohgemisch) [Mol.-%] | 32,0 | 57,0 | 55,0 | 44,0 | 41,0 | 50,0 | 57,0 | 38,0 | 34,0 |

1) $SO_3$-Zugabe erfolgte in Gegenwart Methylenchlorid

2) Die bei der Umsetzung entstandene Sulfonsäure sowie der Sulfonsäureester liegen als cis/trans-Isomerengemisch vor. Eine Auftrennung war nicht möglich

3) Die angegebenen Werte beziehen sich auf die Gewichtsmenge der Einsatzkomponenten

4) Die angegebenen Ausbeutewerte in Mol.-% beziehen sich auf den eingesetzten $\alpha,\beta$-ungesättigten Carbonsäureester

*Beispiel 12*

*Herstellung des Magnesiumsalzes des 2-Sulfo-propensäuremethylesters*

In 16,6 g 2-Sulfo-propensäuremethylester, gelöst in 30 g Acetonitril, werden bei Raumtemperatur 2 g Magnesiumoxid eingerührt. Der nach 4 Stunden vorliegende weisse Feststoff wird durch Filtration abgetrennt und mit Acetonitril gewaschen. Man erhält das Salz der Formel

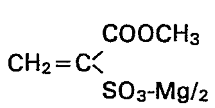

in einer Ausbeute von 17,7 f (100%, bezogen auf die eingesetzte Sulfonsäure)

$^1$H-NMR-Spektrum (in $D_2O$): $\delta$ 3,85 (s), 6,65 (d) ppm

*Beispiel 13*

*Herstellung des Zinksalzes des 2-Sulfo-propensäuremethylesters*

16,6 g 2-Sulfo-propensäuremethylester, gelöst in 30 g Acetonitril, werden unter Rühren bei 15°C mit 4,1 g Zinkoxid umgesetzt. Der nach 5 Stunden entstandene weisse Feststoff wird durch Filtration abgetrennt und mit Acetonitril gewaschen. Man erhält das Salz der Formel

$$CH_2{=}C\begin{smallmatrix} COOCH_3 \\ SO_3\text{-}Zn/_2 \end{smallmatrix}$$

in einer Ausbeute von 18,8 g (95%, bezogen auf die eingesetzte Sulfonsäure

$^1H$-NMR-Spektrum (in $D_2O$): $\delta$ 3,85 (s), 6,65 (d) ppm

*Beispiel 14*

*Umsetzung von Acrylsäuremethylester mit $SO_3$ und Dimethylsulfat*

In eine Vorlage von 253 g Acrylsäuremethylester, stabilisiert mit 2 g Hydrochinon, werden bei 0° bis 10°C unter Einleiten eines schwachen Inertgas-Stromes 235 g Schwefeltrioxid in flüssiger Form eingerührt. Man lässt die Reaktionsmischung auf Raumtemperatur kommen und tropft schnell 740 g Dimethylsulfat zu. Nach 3stündigem Erhitzen des Gemisches auf 160°C werden im Vakuum 480 g leicht flüchtige Bestandteile — überwiegend Dimethylsulfat — abdestilliert. Anschliessend wird das Rohgemisch unter folgenden Bedingungen in einen Dünnschichtverdampfer eingetragen:

| | |
|---|---|
| Temperatur des Heizmantels: | 145°C |
| eingestellter Druck: | 0,5 bis 1,0 mbar |
| Durchsatzmenge: | 1,0 $kg/m^2h$ |

Man erhält ein farbloses Öl.
Ausbeute: 598 g
NMR-spektroskopisch ergibt sich ein Produktverhältnis von:

20% 2-Sulfopropensäuremethylester
80% 2-Methoxysulfonylpropensäuremethylester

Die Reinigung und Trennung des Produktgemisches wird gemäss Beispiel 1 durchgeführt.

Ausbeute: 205 g 2-Methoxysulfonylpropensäuremethylester (42 Gew.-%, bezogen auf die eingesetzten Komponenten Acrylsäuremethylester und $SO_3$)

**Patentansprüche**

1. Verfahren zur Herstellung von Verbindungen der Formel

$$R_1\text{-}CH{=}\underset{\displaystyle SO_3X}{\underset{|}{C}}\text{—}CO_2R_2$$

worin
$R_1$ Wasserstoff oder Methyl,
$R_2$ eine primäre oder sekundäre Alkylgruppe mit 1 bis 4 C-Atomen und
X Wasserstoff, eine primäre oder sekundäre Alkylgruppe mit 1 bis 4 C-Atomen
bedeuten, dadurch gekennzeichnet, dass man $\alpha,\beta$-ungesättigte Carbonsäureester der allgemeinen Formel

$$R_1\text{-}CH{=}CH\text{-}CO_2R_2$$

in der $R_1$ und $R_2$ die obengenannten Bedeutungen haben, bei Temperaturen von —10 bis +25°C mit 0,6 bis 1,6 Moläquivalenten Schwefeltrioxid und gegebenenfalls mit 0,1 bis 10 Moläquivalenten eines Alkylierungsmittels, jeweils bezogen auf den ungesättigten Ester, umsetzt, das Reaktionsgemisch auf Temperaturen von 70 bis 200°C erhitzt, destillativ aufarbeitet und die Komponenten des Destillats gegebenenfalls trennt.

2. Verfahren nach Anspruch 1, dadurch gekennzeichnet, dass man die Umsetzung der $\alpha,\beta$-ungesättigten Carbonsäureester bei 0 bis +10°C mit 0,8 bis 1,2 Moläquivalenten Schwefeltrioxid während 0,1 bis 2,0 Stunden vornimmt.

3. Verfahren nach Anspruch 1, dadurch gekennzeichnet, dass man die Umsetzung in Gegenwart eines organischen Lösungsmittels vornimmt.

4. Verfahren nach Anspruch 1, dadurch gekennzeichnet, dass man aus dem destillativ aufgearbeiteten Reaktionsgemisch die Verbindung, in der X Wasserstoff bedeutet durch Ausfällen mit einem organischen Fällmittel oder durch Salzbildung abtrennt und den verbleibenden Sulfonsäureester destillativ isoliert.

5. Verfahren nach Anspruch 1, dadurch gekennzeichnet, dass man die destillative Aufarbeitung des Reaktionsgemisches, gegebenenfalls nach Abtrennung leicht flüchtiger Bestandteile, in einem Verdampfungsrohr bei Temperaturen von 100 bis 250°C, einem Druck von 0,05 bis 30 mbar und einem Durchsatz von 0,1 bis 10 $kg/m^2.h$ durchführt.

6. Verfahren nach Anspruch 1, dadurch gekennzeichnet, dass man als $\alpha,\beta$-ungesättigten Carbonsäureester Acrylsäuremethylester einsetzt.

7. Verfahren nach Anspruch 1, dadurch gekennzeichnet, dass man 0,2 bis 5 Moläquivalente eines $C_1$-$C_4$-Dialkylsulfates, bezogen auf den $\alpha,\beta$-ungesättigten Carbonsäureester, der Reaktionsmischung zusetzt.

8. Verbindungen der Formel

$$R_1\text{-}CH{=}C\begin{smallmatrix} CO_2R_2 \\ SO_3^{\ominus} \quad X^{\oplus} \end{smallmatrix}$$

worin
$R_1$ Wasserstoff oder Methyl,
$R_2$ eine primäre oder sekundäre Alkylgruppe mit 1 bis 4 C-Atomen und
$X^{\oplus}$ ein Proton oder Metallionäquivalent bedeuten.

9. Verbindungen nach Anspruch 4, worin $X^{\oplus}$ ein Proton oder ein Alkalimetall-, Erdalkalimetall-, Zink- oder Aluminiumionäquivalent bedeutet.

10. Verbindungen nach Anspruch 8, worin $R_1$ Wasserstoff und $R_2$ Methyl bedeuten.

**Claims**

1. Process for the preparation of compounds of the formula

$$R_1\text{-}CH{=}\underset{\displaystyle SO_3X}{\underset{|}{C}}\text{—}CO_2R_2$$

wherein
$R_1$ denotes hydrogen or methyl,
$R_2$ denotes a primary or secondary alkyl group with 1 to 4 C atoms and
X denotes hydrogen, or a primary or secondary alkyl group with 1 to 4 C atoms,
characterised in that $\alpha,\beta$-unsaturated carboxylic acid esters of the general formula

$$R_1\text{-}CH=CH\text{-}CO_2R_2$$

in which $R_1$ and $R_2$ have the above-mentioned meanings, are reacted at temperatures of —10 to +25°C with 0.6 to 1.6 mol. equivalents of sulphur trioxide and, optionally, with 0,1 to 10 mol. equivalents of an alkylating agent, based in each case on the unsaturated ester, the reaction mixture is heated to temperatures of 70 to 200°C and worked up by distillation and the components of the distillate are optionally separated.

2. Process according to claim 1, characterised in that the reaction of the $\alpha,\beta$-unsaturated carboxylic acid esters is carried out at 0 to +10°C with 0.8 to 1.2 mol. equivalents of sulphur trioxide for 0.1 to 2.0 hours.

3. Process according to claim 1, characterised in that the reaction is carried out in the presence of an organic solvent.

4. Process according to claim 1, characterised in that the compound in which X denotes hydrogen is separated off from the reaction mixture worked up by distillation, by precipitation with an organic precipitant or by salt formation and the remaining sulphonic acid ester is isolated by distillation.

5. Process according to claim 1, characterised in that the distillative working up of the reaction mixture is carried out, optionally after separating off readily volatile constituents, in a tubular evaporator at temperatures of 100 to 250°C, under a pressure of 0.05 to 30 mbar and with a throughput of 0.1 to 10 kg/m$^2$.h.

6. Process according to claim 1, characterised in that acrylic acid methyl ester is used as the $\alpha,\beta$-unsaturated carboxylic acid ester.

7. Process according to claim 1, characterised in that 0.2 to 5 mol. equivalents of a $C_1$-$C_4$-dialkyl sulphate, based on the $\alpha,\beta$-unsaturated carboxylic acid ester, are added to the reaction mixture.

8. Compounds of the formula

$$R_1\text{-}CH=C\begin{matrix}CO_2R_2\\ SO_3^{\ominus}\quad X^{\oplus}\end{matrix}$$

wherein
$R_1$ denotes hydrogen or methyl,
$R_2$ denotes a primary or secondary alkyl group with 1 to 4 C atoms and
$X^{\oplus}$ denotes a proton or metal ion equivalent.

9. Compounds according to claim 4, wherein $X^{\oplus}$ denotes a proton or an alkali metal, alkaline earth metal, zinc or aluminium ion equivalent.

10. Compounds according to claim 8, wherein $R_1$ denotes hydrogen and $R_2$ denotes methyl.

**Revendications**

1. Procédé de préparation de composés répondant à la formule

$$R_1\text{-}CH=\underset{\displaystyle SO_3X}{\underset{|}{C}}\text{—}CO_2R_2$$

dans laquelle
$R_1$ représente un hydrogène ou un méthyle,
$R_2$ un groupe alkyle primaire ou secondaire avec 1 à 4 atomes de C et
X un hydrogène, un groupe alkyle primaire ou secondaire avec 1 à 4 atomes de C,
caractérisé en ce que l'on fait réagir des esters d'acides carboxyliques $\alpha,\beta$-insaturés de formule générale

$$R_1\text{-}CH=CH\text{-}CO_2R_2$$

dans laquelle $R_1$ et $R_2$ ont les significations indiquées précédemment, à des températures de —10°C à +25°C avec 0,6 à 1,6 équivalents molaires d'anhydride sulfurique et éventuellement avec 0,1 à 10 équivalents molaires d'un agent d'alkylation, rapportés chaque fois à l'ester insaturé, que l'on chauffe le mélange réactionnel pendant 0,5 à 6 heures à des températures de 70°C à 200°C, le traite par voie de distillation et sépare éventuellement les constituants du distillat.

2. Procédé selon la revendication 1, caractérisé en ce qu'on effectue la réaction des esters d'acides carboxyliques $\alpha,\beta$-insaturés entre 0 et +10°C avec 0,8 à 1,2 équivalents molaires d'anhydride sulfurique pendant 0,1 à 2 heures.

3. Procédé selon la revendication 1, caractérisé en ce qu'on effectue la réaction en présence d'un solvant organique.

4. Procédé selon la revendication 1, caractérisé en ce qu'on sépare du mélange réactionnel traité par voie de distillation le composé dans lequel X représente un hydrogène, par précipitation avec un précipitant organique ou par salification et que l'on isole l'ester d'acide sulfonique restant par distillation.

5. Procédé selon la revendication 1, caractérisé en ce que l'on effectue le traitement distillatoire du mélange réactionnel, éventuellement après séparation des constituants volatils, dans un tube d'évaporation à des températures de 100 à 250°C, une pression de 0,05 à 30 mbars et avec un débit de 0,1 à 10 kg/m$^2$.h.

6. Procédé selon la revendication 1, caractérisé en ce que l'on met en œuvre de l'acrylate de méthyle en tant qu'ester d'acide carboxylique $\alpha,\beta$-insaturé.

7. Procédé selon la revendication 1, caractérisé en ce qu'on ajoute au mélange réactionnel 0,2 à 5 équivalents molaires d'un sulfate de dialkyle en $C_1$ à $C_4$, par rapport à l'ester d'acide carboxylique $\alpha,\beta$-insaturé.

8. Composés répondant à la formule

$$R_1\text{-}CH=C\begin{matrix}CO_2R_2\\ SO_3^{\ominus}\quad X^{\oplus}\end{matrix}$$

dans laquelle

$R_1$ représente un hydrogène ou un méthyle,

$R_2$ un groupe alkyle primaire ou secondaire avec 1 à 4 atomes de carbone et

$X^{\oplus}$ un proton ou un équivalent d'ion métallique.

9. Composés selon la revendication 4, caractérisés en ce que $X^{\oplus}$ représente un proton ou un équivalent d'ion de métal alcalin, de métal alcalino-terreux, d'ion zinc ou aluminium.

10. Composés selon la revendication 8, caractérisés en ce que $R_1$ représente un hydrogène et $R_2$ un méthyle.